# EUROPEAN PATENT APPLICATION

(11) **EP 1 813 938 A1**
(43) Date of publication of application: **01.08.2007**
(21) Application number: 06001590.6
(22) Date of filing: 26.01.2006
(51) Int. Cl.: G01N 27/447

(54) **Composition for an electrophoresis matrix**

(71) Applicant: GeneCraft GmbH, 59348 Lüdinghausen (DE)
(72) Inventor: Ignatov, Konstantin, 117246 Moscow (RU); Kramarov, Vladimir, 113303 Moscow (RU); Plachov, Dimitrij, 59348 Lüdinghausen (DE)
(74) Representative: Von Renesse, Dorothea

(57) **Abstract**

A composition for preparing an electrophoresis matrix comprising at last one gelling polymer and a staining dye.

## Description

### Field of the Invention

The present invention relates to improvements in methods of gel electrophoresis e.g. for nucleic acids. In particular, it provides improvements of electrophoretic resolution and safety of electrophoretic procedures.

### Background to the Invention

Gel electrophoresis of nucleic acids through agarose or polyacrylamide gels is the standard method used to separate, identify, and purify nucleic acids, e.g. DNA and RNA fragments. This technique is very important for basic biological research, biomedical research and biotechnology industry. Procedures of DNA and RNA gel electrophoresis are well known in the arts and described in detail (Sambrook et al., (1989) "Molecular cloning - A Laboratory Manual", Chapters 6 and 7, Cold Spring Harbor Laboratory Press).

The location of the nucleic acid within the gel is determined directly by staining the gel with fluorescent dyes that specifically bind to nucleic acids and thus detect hem (Sambrook et al.). For staining nucleic acids within the gel various techniques are known to the skilled person, for example dyes such as cyanine dyes (e.g. SYBR Gold, SYBR Green, PicoGreen, OliGreen, RiboGreen, TO-PRO, YO-PRO, PO-PRO, SYTOX, POPO, BOBO, YOYO, TOTO, JOJO, LOLO and other dyes), phenanthridines (e.g. ethidium bromide, propidium iodide, ethidium homodimer, hexidium iodide, dihydroethidium and other dyes), acridines (e. g. acridine orange, acridine homodimer, proflavine, 9-amino-6-chloro-2-methoxyacridine and others), bisbenzimide dyes (e.g. Hoechst 33258, Hoechst 33342 and Hoechst 34580), phenylindole dyes (e.g. 4',6-diamidino-2-phenylindole), and other stains (e.g. Nissl stains, LDS 751, hydroxystilbamidine, 7-aminoactinomycin D and actinomycin D) can be used. The said nucleic acid-binding dyes are available from various commercial sources such as Molecular Probes, Inc. and other companies.

The prevalent dyes for staining nucleic acids within gels are propidium iodide ("Reagent for the fluorescent staining of nucleic acids", J. Tas et al. (1981), Cytochem. 29, p.929), acridine orange ("Versatile probe of nucleic acids and other cell constituents", Z. Darzynkiewicz et al., Flow Cytometry and Sorting (2nd Edit.) p 291 (1990) Wiley-Liss), SYBR Gold and SYBR Green, and the most popular dye - ethidium bromide ("Intercalating agent and fluorescent label for DNA", Review: W. Warring, Antibiotics III 141 (1975), Springer Verlag) (Sambrook et al., (1989) "Molecular cloning - A Laboratory Manual", Chapters 6 and 7, Cold Spring Harbor Laboratory Press).

The staining of macromolecules in gels requires the preparation and utilization of dye-containing buffer solutions in large quantities. Unfortunately, the property of the above dyes to bind to nucleic acids makes most of them carcinogens and mutagens, since they interact with nucleic acids in cells and therewith disturb DNA replication (e.g. ethidium bromide, propidium iodide, acridine orange, proflavine, SYBR Green, actinomycin D and others). Their presence significantly increases the risk of defective copies. With an increasing number of defects, the risk of the development of cells with a defective regulation of the cellular cycle increases. These cells cause cancer. Thus, there are two main problems when using dyes for staining macromolecules: laboratory safety and disposal/detoxification of laboratory solutions containing dyes.

For increasing the research safety, protective measures (such as wearing gloves, goggle, overall) are used. During electrophoresis procedures these measures reduce the risk skin contamination with the dyes.

For decontamination of solutions containing nucleic acids staining dyes, a few methods are used (Sambrook et al., (1989) "Molecular cloning - A Laboratory Manual", pp. 6.16 - 6.17, Cold Spring Harbor Laboratory Press). The prevalent methods of decontamination are based on adsorption of the dyes using adsorbents such as Amberlite and Charcoal ("Ethidium bromide: Destruction and decontamination of solutions", G. Lunn and E.B. Sansone, (1987), Anal. Biochem. 162, p. 453) ("Ethidium bromide and safety", O. Bensaude, (1988), Trends Genet. 4, p.89).

A wide application of gel electrophoresis of nucleic acids needs an improvement of safety during performing electrophoresis, and a considerable simplification of buffer solutions decontamination or a technique for preventing contamination of the solutions.

### Disclosure of the Invention

It is the objective of the present invention to provide means for the improvement of methods of electrophoresis. This problem is solved by providing a composition comprising a gelling polymer or mixtures of polymers containing nucleic acid staining dyes. Most preferably the dye is bound to the polymer. In particular, the invention provides a composition comprising gelling polymer(s) and a dye, wherein said dye is covalently bound to the polymer.

The term "gelling polymer" as used for the present invention means any polymer or a mixture of polymers which can be used to constitute a gel matrix for electrophoresis or parts thereof. Gelling polymers and mixtures thereof are known to the skilled person. Preferred polymers for forming gel matrix are agarose, polyacrylamide or a mixture of agarose and polyacrylamide. Preparation of gels from said polymers has been described in detail (Sambrook et al., (1989) "Molecular cloning - A Laboratory Manual", Chapters 6 and 7, Cold Spring Harbor Laboratory Press).

The terms "dye" or "staining dye" or "binding dye" are used interchangeable and are defined as any dye which can be used to stain a macromolecule within a gel matrix. Most preferably the dye specifically interacts with the macromolecule therewith enabling a specific and selective detection of the macromolecule within the gel matrix. Preferred dyes for the present invention are cyanine dyes (e.g. SYBR Gold, SYBR Green, PicoGreen, OliGreen, RiboGreen, TO-PRO, YO-PRO, PO-PRO, SYTOX, POPO, BOBO, YOYO, TOTO, JOJO, LOLO and other dyes), phenanthridines (e.g. ethidium bromide, propidium iodide, ethidium homodimer, hexidium iodide, dihydroethidium and other dyes), acridines (e. g. acridine orange, acridine homodimer, proflavine, 9-amino-6-chloro-2-methoxyacridine and others), bisbenzimide dyes (e.g. Hoechst 33258, Hoechst 33342 and Hoechst 34580), phenylindole dyes (e.g. 4',6-diamidino-2-phenylindole), and other stains (e.g. Nissl stains, LDS 751, hydroxystilbamidine, 7-aminoactinomycin D and actinomycin D) can be used.

The term "macromolecule" as used herein is defined as any molecule, which is to be detected by using electrophoresis. A preferred macromolecule is a nucleic acid. A nucleic acid is any sequence of nucleotides, either of natural, synthetic or recombinant origin; comprising RNA or DNA or mixtures thereof.

According to one preferred embodiment of the invention the dye is bound to the polymers of the gel matrix. This bound can be any chemical or physical interaction between the dye and the polymer which results in a fixation of the dye on the polymer. Therewith, preferably, an elution of the dye from the gel matrix - during or after the preparation of the gel matrix from the gelling polymers - is avoided or significantly reduced. In a particularly preferred embodiment the dye is covalently bound to gel matrix molecules, therewith being not able to diffuse from the gels and to contaminate buffer solutions or skin of laboratory personal.

It is the advantage of the present invention to improve safety in laboratory procedures by using the composition of the invention. Herewith the handling of staining dye buffers and solution can be avoided and the risk of contamination of the human with the carcinogen dye is minimized.

In another aspect, the invention provides for a higher resolution in nucleic acid gel electrophoresis. The usage of gels, in which a nucleic acid staining dye is covalently bound with a gel matrix, provides a better electrophoretic resolution of nucleic acids than usage of conventional gels. The better resolution is achieved by interaction between the dye, which is covalently bound to the gel matrix, and nucleic acid molecules.

The present invention provides compositions and reagents and kits for performing nucleic acids electrophoresis through gels containing nucleic acids staining dyes which are covalently bound with gel matrix molecules.

In a preferred embodiment the gel electrophoresis with the composition of the invention is an electrophoresis of dsDNA, ssDNA, ssRNA, dsRNA or RNA-DNA hybrid molecules or a mixture of two or more of them through a gel.

The nucleic acid staining dye, which is covalently bound with a gel matrix, can be a fluorescent nucleic acid staining dye. This dye can be selected from cyanine dyes, phenanthridine dyes, acridine dyes, bisbenzimide dyes, phenylindole dyes, or another group of dyes.

The kits of the invention for performing gel electrophoresis of nucleic acids can comprise a ready-made gel containing the dye covalently bound with a polymer forming the gel matrix, or they can comprise reagents for preparation of the said gel. Furthermore, the kit of the invention may contain a plurality of additional components such as buffer solutions or reagents for preparation of buffer solutions.

Other features, aspects and advantages of the invention will be, or will become, apparent to one with skill in the art upon examination of the following figures and detailed description. It is intended that all such additional systems, features, aspects and advantages included within this description are within the scope of the invention, and are protected by the following claims.

The kit according to the invention may comprise a ready to use gel containing a nucleic acid staining dye covalently bound with the polymer forming the gel matrix, or it may comprise reagents for preparation of the said ready gel such as agarose with covalently bound dye (e.g. ethidium bromide). Furthermore, the kit of the invention may contain a plurality of additional components such as buffer solutions or reagents for preparation of buffer solutions.

One preferred embodiment of the present invention relates to improvements of electrophoretic resolution and safety in nucleic acid gel electrophoresis through the use of gels containing nucleic acid staining dyes, which are covalently bound to polymer molecules that form the gel matrix ("gelling polymer").

The improvement of safety in the laboratory procedures and the prevention of contamination are achieved by using gels in which nucleic acid detecting dyes (e.g. ethidium bromide or proflavine) are covalently bound with polymer molecules that form the gel matrix. The dyes, which are covalently bound to gel matrix molecules, are not able to diffuse from the gels and to contaminate electrophoretic solutions or skin of the worker. In addition, the method of the invention does not require any use of solutions containing the dye for staining the gel or prevention of dye diffusion from the gel.

The improvement of the electrophoretic resolution is also a result of the fixation of the dye to the polymer matrix of the gel, preferably by a covalent bound. These dyes are preferably nucleic acid-binding dyes, and when covalently bound to gel matrix, they increase interaction between nucleic acid molecules and the gel matrix. The increased interaction between nucleic acids and the gel matrix provides a better separation of nucleic acid molecules and reduces the diffusion of nucleic acid bands during electrophoresis. As a result, electrophoretic resolution of nucleic acids is improved.

In a preferred aspect, the gel matrix for the gel electrophoresis is formed by polymer molecules covalently bound to a nucleic acid staining dye. Preferred polymers for forming gel matrix are agarose, polyacrylamide or a mixture of agarose and polyacrylamide. Preparation of gels from said polymers has been described in detail (Sambrook et al., (1989) "Molecular cloning - A Laboratory Manual", Chapters 6 and 7, Cold Spring Harbor Laboratory Press).

A preferred nucleic acid staining dye, covalently bound with a gel matrix, can be a fluorescent nucleic acid staining dye. This fluorescent dye can be from cyanine dyes, phenanthridine dyes, acridine dyes, bisbenzimide dyes, phenylindole dyes, or another group of dyes.

Preferred nucleic acid staining dyes from cyanine dyes are SYBR Gold, SYBR Green, PicoGreen, OliGreen, RiboGreen, TO-PRO, YO-PRO, PO-PRO, SYTOX, POPO, BOBO, YOYO, TOTO, JOJO and LOLO dyes.

Preferred nucleic acid staining dyes from phenanthridine dyes are ethidium bromide, propidium iodide, ethidium homodimer, hexidium iodide and dihydroethidium.

Preferred nucleic acid staining dyes from acridine dyes are acridine orange, acridine homodimer, proflavine, 9-amino-6-chloro-2-methoxyacridine.

Preferred nucleic acid staining dyes from bisbenzimide dyes are Hoechst 33258, Hoechst 33342 and Hoechst 34580.

Preferred nucleic acid staining dye from phenylindole dyes is 4',6-diamidino-2-phenylindole.

Other preferred nucleic acid staining dyes are Nissl stains, LDS 751, hydroxystilbamidine, 7-aminoactinomycin D and actinomycin D.

All of the above mentioned dyes are commercially available from Molecular Probes, Inc. The names of the said cyanine dyes (SYBR Gold, SYBR Green, PicoGreen, OliGreen, RiboGreen, TO-PRO, YO-PRO, PO-PRO, SYTOX, POPO, BOBO, YOYO, TOTO, JOJO and LOLO) are trademarks of Molecular Probes, Inc. The following dyes are also commercially available from Fluka, Sigma-Aldrich and other companies: ethidium bromide, propidium iodide, ethidium homodimer, acridine orange, acridine homodimer, proflavine, Hoechst dyes, 4',6-diamidino-2-phenylindole, Nissl stains, hydroxystilbamidine, 7-aminoactinomycin D and actinomycin D.

As indicated above, the gels of the present invention contain a nucleic acid staining dye which is covalently bound to molecules of the gel matrix.

In one embodiment, the dye, which may be selected from the group consisting of ethidium bromide, propidium iodide, ethidium homodimer, acridine orange, acridine homodimer, proflavine, SYBR Gold, RiboGreen and SYBR Green, is covalently bound to molecules of agarose.

In another embodiment, the dye, which may be selected from the group consisting of ethidium bromide, propidium iodide, ethidium homodimer, acridine orange, acridine homodimer, proflavine, SYBR Gold, RiboGreen and SYBR Green, is covalently bound with molecules of polyacrylamide.

In a preferred embodiment, the dye is selected from the group consisting of ethidium bromide, propidium iodide, acridine orange, proflavine, SYBR Gold and SYBR Green, and covalently bound to molecules of agarose.

The dye can be covalently bound to the polymer before, during or after preparing the gel matrix.

In one embodiment, the dye is covalently bound to the polymer, preferably agarose, before preparing the gel matrix.

In another embodiment, the dye is covalently bound to the polymer, preferably polyacrylamide, during or after preparing the gel matrix.

In a preferred embodiment, the dye, selected from the group consisting of ethidium bromide, propidium iodide, acridine orange, proflavine, SYBR Gold and SYBR Green, is covalently bound with the polymer, preferably molecules of agarose, before the preparation of the gel matrix.

The dyes can be covalently bound with the polymers by different methods. These methods are well known in the art and widely used for preparation of chromatographic sorbents. A number of these methods are described in detail ["Chromatography of proteins and nucleic acids" L.A. Osterman, pp. 339-398; Nauka Publishers, Moscow (1985)].

In a preferred embodiment, a nucleic acid staining dye may be covalently bound with a polymer forming a gel matrix through -NH₂, -NH-, -OH or -SH groups of dye molecules.

Preferred chemical reactions for binding dye molecules to a polysaccharide polymer forming a gel matrix, such as agarose, can be the following:
1) Reactions with bromocyanogen 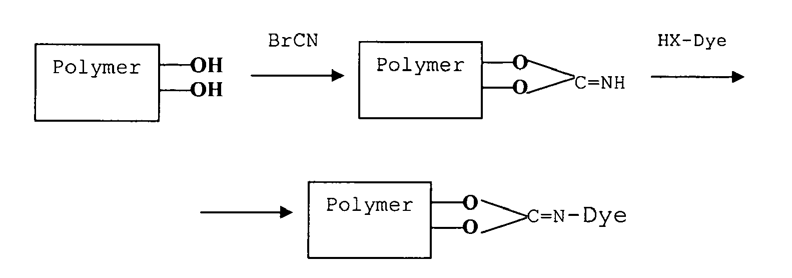 wherein (HX-) may be (HS-), (HO-), (H₂N-), (HRN-) or (HN=).
2) Reactions with epichlorohydrin 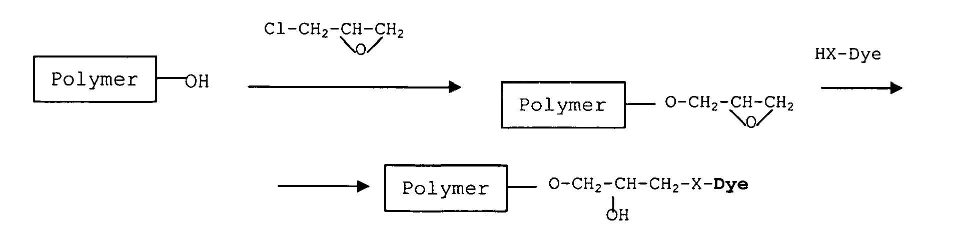 wherein (-X-) may be (-S-), (-O-), (-NH-), (-NR-) or (-N=).
3) Reactions with trichlorotriazine 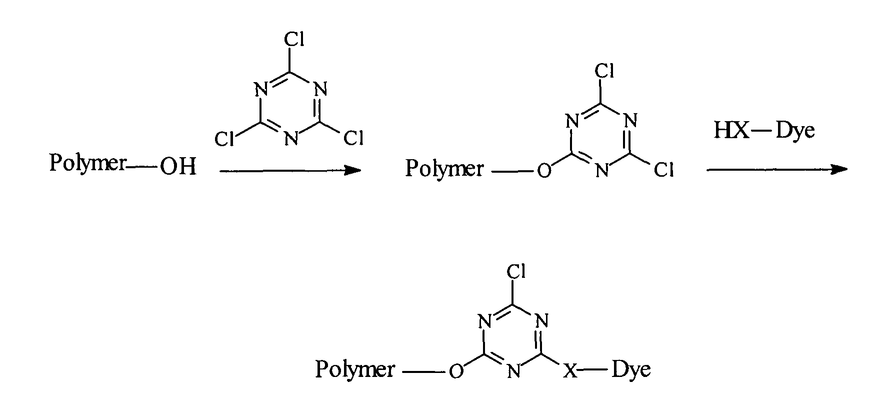 wherein (-X-) may be (-S-), (-O-), (-NH-), (-NR-) or (-N=).
4) Reactions with quinone 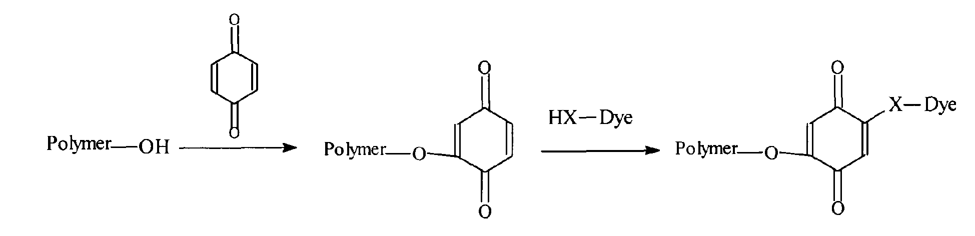
wherein (-X-) may be (-S-), (-O-), (-NH-), (-NR-) or (-N=).

Preferred chemical reactions for binding dye molecules to gel matrix polymer molecules containing amide groups, such as polyacrylamide, may be reactions with glutaraldehyde: 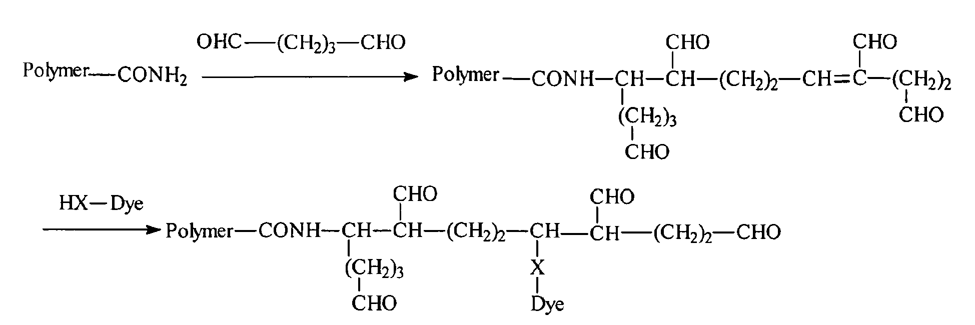 wherein (-X-) may be (-S-), (-O-), (-NH-), (-NR-) or (-N=).

### Examples

The following Examples illustrate aspects of the invention. The Examples are illustrative of, but not binding on, the present invention. Any methods, preparations, solutions and such like, which are not specifically defined, may be found in Sambrook et al. All solutions are aqueous and made up in sterile, deionised water, unless otherwise specified. All reagents were obtained from the Sigma-Aldrich Corporation.

### Brief Description of the Figures

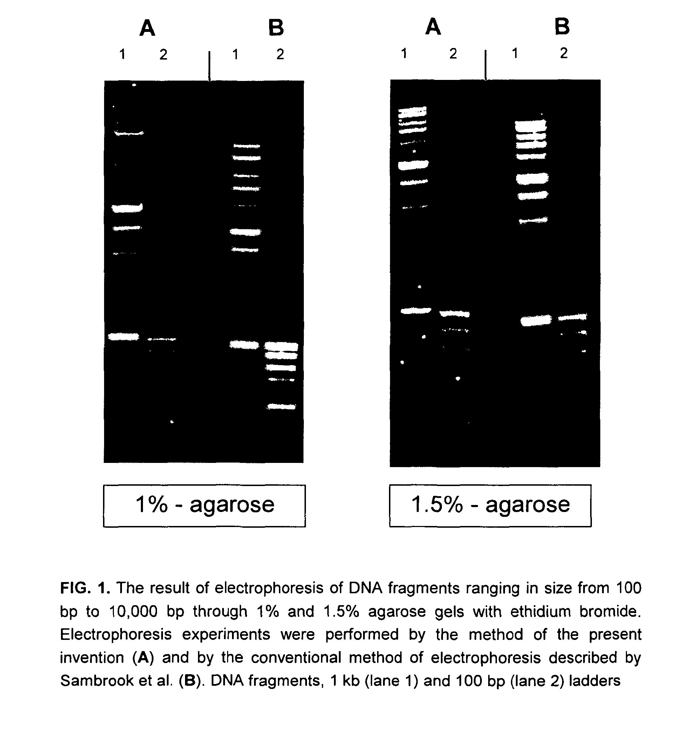

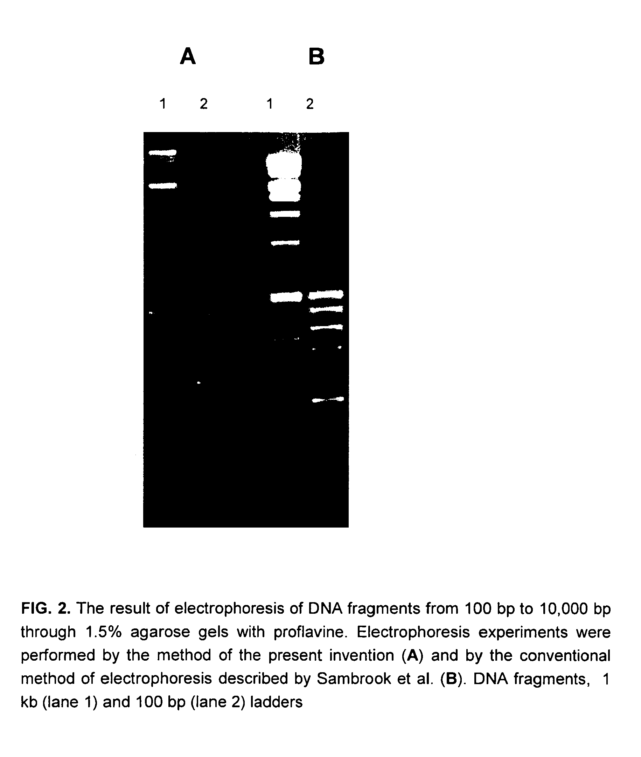

FIG. 1 and FIG. 2 depict the results of electrophoreses of DNA fragments ranging in size from 100 bp to 10,000 bp, which were performed by the conventional method of electrophoresis described in Sambrook et al. and the method of the present invention.

### Example 1

### Preparation of agarose containing ethidium bromide covalently bound to agarose molecules.

### A) Preparation of chemically activated agarose.

For the preparation of chemically activated agarose quinone was used.

Agarose (10 g) was suspended in 100 ml of 0.1 M phosphate buffer (pH=8.0) containing 20% ethanol.

Quinone (0.2 g) was dissolved in 20 ml of ethanol and added to the suspension of agarose. The suspension was then held at room temperature for 1 hour.

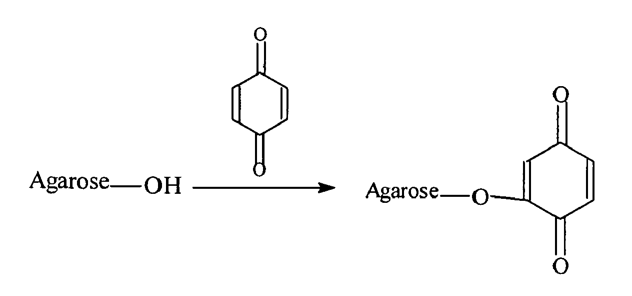

### B) Chemical immobilization of ethidium bromide

The obtained chemically activated agarose was washed with 20% ethanol and suspended in 100 ml of 0.1 M phosphate buffer (pH=8.0) containing 20% ethanol and 0.1 g of ethidium bromide. Then the suspension of agarose was held at 4°C for 24 hours.

The obtained agarose, containing covalently bound ethidium bromide, was washed with 0.1 M Na-acetate (pH=4.0) and then with 50% ethanol. The washed agarose was dried and used for agarose gel preparation.

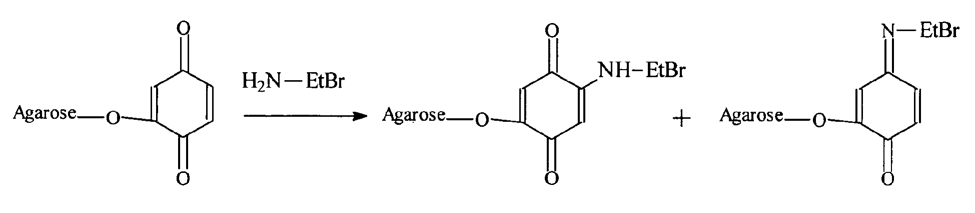

### Example 2

### Preparation of agarose containing proflavine covalently bound to molecules of agarose.

Preparation of chemically activated agarose was performed as described in Example 1.

The obtained chemically activated agarose was washed with 20% ethanol and suspended in 100 ml of 0.1 M phosphate buffer (pH=8.0) containing 20% of ethanol and 0.25 g of proflavine hemisulfate. Then the suspension of agarose was held at 4°C for 24 hours.

The obtained agarose, containing covalently bound proflavine, was washed with 0.1 M Na-acetate (pH=4.0) and then with 50% ethanol. The washed agarose was dried and used for preparation of agarose gel.

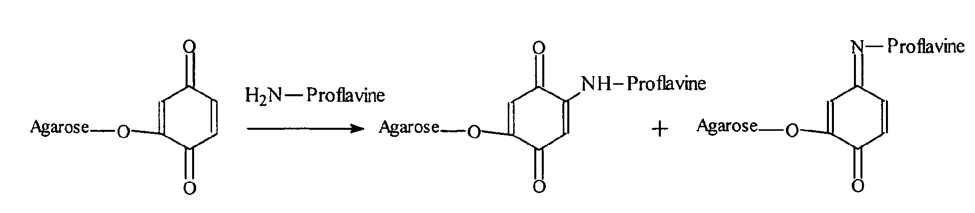

### Example 3

### Gel electrophoresis of DNA through agarose gel containing ethidium bromide which is covalently bound to the agarose gel matrix.

For gel preparation, agarose obtained in Example 1 was used. The 1% and 1.5% agarose gels and electrophoretic buffers were prepared as described by Sambrook et al. in chapter 6, with the exception of addition of ethidium bromide or other dyes to gels and buffer solutions.

The procedure of agarose electrophoresis of DNA fragments ranging in size from 100 bp to 10,000 bp was performed as described by Sambrook et al. in chapter 6.

After the electrophoresis, the contamination of the electrophoretic buffer solutions was controlled by fluorescent analysis and it was not detected.

In parallel, conventional agarose electrophoresis of the same DNA fragments was performed. In this case, 1% and 1.5% agarose gel and electrophoretic buffers were prepared with the addition of ethidium bromide to gel and buffer solutions, as described by Sambrook et al.

**FIG. 1** depicts results of the electrophoresis. Compared to the conventional method of electrophoresis described by Sambrook et al., the method of the invention provides better electrophoretic resolution of the DNA bands, and at the same time the invention method prevents contamination of the electrophoretic solutions with ethidium bromide and improves the safety of the electrophoretic procedure.

### Example 4

### Gel electrophoresis of DNA through agarose gel containing proflavine which is covalently bound to the agarose gel matrix.

For the preparation of agarose gel containing covalently bound proflavine agarose obtained in Example 2 was used. The 1.5% agarose gel and electrophoretic buffers were prepared as described by Sambrook et al. with the exception of any addition of proflavine or other dyes to gels and buffer solutions.

The procedure of agarose electrophoresis of DNA fragments ranging in size from 100 bp to 10,000 bp was performed as described by Sambrook et al.

After the electrophoresis, the contamination of the electrophoretic buffer solutions was controlled by fluorescent analysis and it was not detected.

In parallel, ordinary agarose electrophoresis of the same DNA fragments was performed. In this case, 1.5% agarose gel and electrophoretic buffers were prepared with the addition of proflavine to the gel and buffer solutions.

**FIG. 2** depicts results of electrophoreses. Compared to the conventional method of electrophoresis, the method of the invention provides better electrophoretic resolution of the DNA bands, and at the same time, the invention method prevents contamination of the electrophoretic solutions with ethidium bromide and improves the safety of the electrophoretic procedure.

## Claims

1. A composition for preparing an electrophoresis matrix comprising at last one gelling polymer and a staining dye.

2. A composition according to claim 1 wherein the dye is covalently bound to the polymers of the gel matrix.

3. A composition according to claims 1 or 2, wherein said dye is covalently bound.

4. A composition according to any of the claim 1 to 3, wherein the dye is a nucleic acid dye.

5. A composition of claim 4 wherein said dye is selected fomr the group of cyanine dye, phenanthridine dye, acridine dye, bisbenzimide dye, phenylindole dye, Nissl stain, LDS 751, hydroxystilbamidine, 7-aminoactinomycin D or actinomycin D.

6. A composition of claim 5, wherein said cyanine dye is SYBR Gold, SYBR Green, PicoGreen, OliGreen, RiboGreen, TO-PRO, YO-PRO, PO-PRO, SYTOX, POPO, BOBO, YOYO, TOTO, JOJO or LOLO dye.

7. A composition of claim 5, wherein said phenanthridine dye is ethidium bromide, propidium iodide, ethidium homodimer, hexidium iodide or dihydroethidium.

8. A composition of claim 5, wherein said acridine dye is acridine orange, acridine homodimer, proflavine or 9-amino-6-chloro-2-methoxyacridine.

9. A composition of claim 5, wherein said bisbenzimide dye is Hoechst 33258, Hoechst 33342 or Hoechst 34580.

10. A composition of claim 5, wherein said phenylindole dye is 4',6-diamidino-2-phenylindole

11. A composition according to any of the above claims, wherein said polymer is selected from agarose or polyacrylamide or a mixture thereof.

12. A kit for preparing an electrophoresis matrix comprising at last one gelling polymer and a staining dye.

13. A kit according to claim 12 comprising a dye according to at least one of the above claims 2 to 12.
